# EUROPEAN PATENT SPECIFICATION

(11) **EP 3 103 496 B1**
(45) Date of publication and mention of the grant of the patent: **15.08.2018**
(21) Application number: 16157128.6
(22) Date of filing: 24.02.2016
(51) Int. Cl.: A61M 11/00, A61M 15/00, B05B 12/08, H02J 7/00, B05B 17/00, G06F 1/32, A61M 16/00, A61M 16/06

(54) **NEBULIZATION SYSTEM**
VERNEBELUNGSSYSTEM
SYSTÈME DE NÉBULISATION

(30) Priority: 11.06.2015 US 201562174144 P; 30.09.2015 TW 104132036
(43) Date of publication of application: 14.12.2016
(73) Proprietor: Delta Electronics, Inc., Taoyuan County 33341 (TW)
(72) Inventor: LIN, Jung-Yu, 33341 Taoyuan City (TW); MA, I-Chen, 33341 Taoyuan City (TW); HSIEH, Hung-Chia, 33341 Taoyuan City (TW); SHIH, Yu-Jia, 33341 Taoyuan City (TW); PAI, Sheng-Wen, 33341 Taoyuan City (TW)
(74) Representative: dompatent von Kreisler Selting Werner - Partnerschaft von Patent- und Rechtsanwälten mbB

(56) References cited:
- DE-A1- 2 820 951
- US-A1- 2007 019 442
- US-A1- 2009 192 443
- US-A1- 2011 203 580
- US-A1- 2013 293 018
- US-A1- 2015 128 976

## Description

### BACKGROUND

### Technical Field

The invention relates to a nebulization system and a nebulizer.

### Related Art

Recently, various nebulizers namely sprayers are widely applied to healthcare or beauty care. They nebulize medicated liquid, lotion or essence, etc. by nebulization devices into a fine mist or aerosol absorbed into a body easily. For example, they can be applied to health care or treatment of respiratory diseases, or faster absorption of medicated liquid or lotion by skin, or aroma, etc.

Practically, the nebulizer can connect to mains electricity which acts as the power source so as to supply drugs at full capacity and shorten the treatment time. When the user utilizes the nebulizer at a place where mains electricity is not available, a battery can be taken for the nebulizer as the power source for supplying drugs. However, due to limited quantity of electricity provided by the battery, if the nebulizer still keeps supplying drugs at full capacity, the battery will quickly run low, the supply time will be also shorten and the course of treatment is blocked.

US 2015/0128976 describes a nebulizer as electronic cigarette which can be connected to mains-electricity or alternative can be driven by a battery unit.

Further, nebulizer are described in DE 28 20 951, US 2011/0203580 and US 2009/0192443 which can be all connected to mains-electricity and/or comprise a battery unit as power source where main-electricity is not available.

In other sides, because the current nebulizer cannot automatically adjust the drug supply with the user's breath rhythm during spray treatment. When breathing out, users would blow medicated mist out and do not indeed inhale it. Thus, the drug is wasted and the treatment effect is influenced.

US 2013/0293018 describes a power supply system adapted to prevent an electronic device from crashing due to an unstable voltage in the mains-electricity. Therefore, a city power detecting unit is employed to detect any anomaly in the mains-electricity. If, by the city power detecting unit a power anomaly is detected, a control unit switches the power supply from the mains-electricity to a battery unit.

Further, US 2007/0019442 describes a power converter based on known bucking circuit which can convert an input voltage in a two-stage conversion to an output voltage.

Therefore, it is needed to provide a nebulization system, a nebulizer and a driving method which can have better performance and battery life, and adjust the drug supply with the user's breath rhythm so as to avoid a waste of medicated liquid.

### SUMMARY

An aspect of the disclosure is to provide a nebulization system and a nebulizer which can have better performance and battery life, and adjust the drug supply with the user's breath rhythm so as to avoid a waste of medicated liquid. The invention is defined by the appended claim 1.

A nebulization system comprises a power adapter and a nebulizer. The nebulizer is adapted to be detachably connected to the power adapter and comprises a nebulization module and a nebulization driving device. The nebulization module has an accommodation room for placing a liquid. The nebulization driving device connects to the nebulization module and comprises a battery unit, an input terminal, a voltage detection unit, a voltage adjustment unit, a control unit and a driving unit. The input terminal is coupled to the power adapter and the battery unit. The voltage detection unit is coupled to the input terminal to detect whether an input voltage of the input terminal is larger than a predefined value. The predefined value is set larger than a voltage outputted from the battery unit. The voltage adjustment unit is coupled to the input terminal. The control unit is coupled to the voltage detection unit and the voltage adjustment unit. The control unit controls the voltage adjustment unit to adjust the input voltage to a first operating voltage as an output voltage if the input voltage is larger than the predefined value, the control unit controls the voltage adjustment unit to adjust the input voltage to a second operating voltage as an output voltage if the input voltage is smaller than the predefined value, and the first operating voltage is larger than the second operating voltage. The driving unit is coupled to the voltage adjustment unit, and drives the nebulization module to nebulize the liquid according to the output voltage of the voltage adjustment unit.

In one embodiment, the nebulization driving device further comprises a PWM unit coupled to the driving unit and generating a PWM signal to control a duty cycle of the driving unit to drive the nebulization module to nebulize the liquid.

In one embodiment, the nebulization driving device further comprises a switch unit having one terminal coupled to the input terminal and having the other terminal coupled to the battery unit. The control unit controls the switch unit to cut off so the battery unit does not supply power to the input terminal if the input voltage is larger than the predefined value.

In one embodiment, the switch unit comprises a switch and a diode, one terminal of the switch is coupled to the battery unit and the anode of the diode, another terminal of the switch is coupled to the input terminal and the cathode of the diode, and the other terminal of the switch is coupled to the control unit.

In one embodiment, the nebulization system further comprises an auxiliary element including a respiration detection unit coupled to the control unit to detect the breath state of a user. The control unit controls the driving unit to act if the user breathes in, and the control unit controls the driving unit to stop acting if the user breathes out.

In one embodiment, the respiration detection unit is a micro switch, an infrared detector or an audio detector.

In one embodiment, the predefined value is smaller than a voltage outputted from the power adapter.

In one embodiment, the nebulizer further comprises a current detection unit coupled to the input terminal to detect an input current corresponding to the input voltage. The control unit calculates electric power according to the input voltage and the input current.

In one embodiment, if the nebulizer adjusts the input voltage to the first operating voltage, the input terminal is disconnected from the battery unit so the
battery unit does not supply electrical power to the input terminal.

In one embodiment, a duty cycle of the nebulizer is controlled by a PWM signal while the input voltage is adjusted to the first operating voltage or the second operating voltage.

As mentioned above, the voltage or current of the input terminal is detected to accordingly determine whether the nebulizer is supplied with mains electricity or battery. Moreover, the driving mode is adjusted according to the power source so as to keep better performance and battery life. In some embodiments, the breath detection unit is utilized to detect the breath state of the user so that the nebulizer can adjust the drug supply with the user's breath rhythm so as to avoid a waste of medicated liquid.

### BRIEF DESCRIPTION OF THE DRAWINGS

The embodiments will become more fully understood from the detailed description and accompanying drawings, which are given for illustration only, and thus are not limitative of the present invention, and wherein:
FIG. 1A is a block diagram of a nebulization system according to the first embodiment;
FIG. 1B is a schematic diagram showing the structure of the nebulization system in FIG. 1A;
FIG. 1C is an exploded diagram showing the nebulizer;
FIG. 2 is a schematic diagram showing the circuit of the switch unit.
FIG. 3A is a waveform diagram showing the output voltage of the driving unit;
FIG. 3B and FIG. 3C are waveform diagrams showing another output voltage of the driving unit;
FIG. 4A is a block diagram of a nebulization system according to the second embodiment;
FIG. 4B is a schematic diagram showing the structure of the nebulization system in FIG. 4A;
FIG. 4C is a schematic diagram showing the auxiliary element;
FIG. 5 is a waveform diagram showing the breath cycle of the user and the output voltage of the driving unit in smart driving mode;
FIG. 6A is a block diagram of a nebulization system according to the third embodiment;
FIG. 6B is a block diagram of a nebulization system according to another embodiment;
FIG. 7 is a flow chart showing steps of the driving method for a nebulization system according to the embodiment;
FIG. 8A is a circuit diagram of the voltage detection unit;
FIG. 8B is a circuit diagram of the voltage adjustment unit and the driving unit;
FIG. 8C and FIG. 8D are circuit diagrams of the driving unit according to other examples; and
FIG. 8E is a circuit diagram of the current detection unit.

### DETAILED DESCRIPTION OF THE INVENTION

The embodiments of the invention will be apparent from the following detailed description, which proceeds with reference to the accompanying drawings, wherein the same references relate to the same elements.

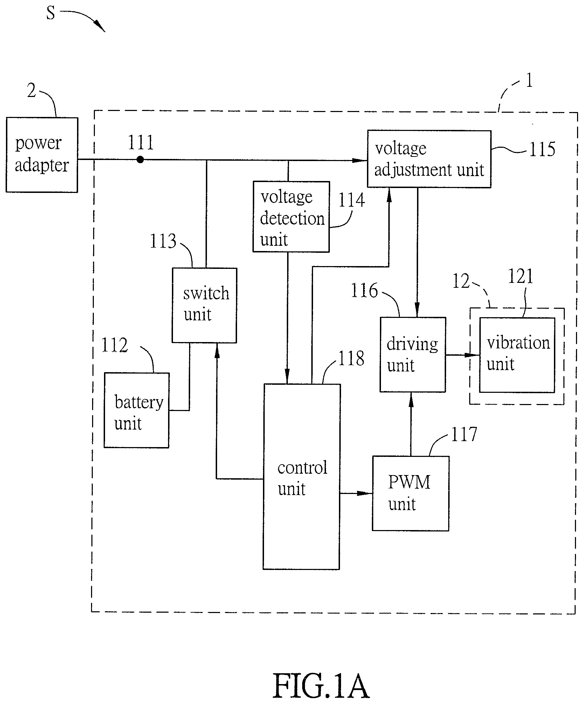

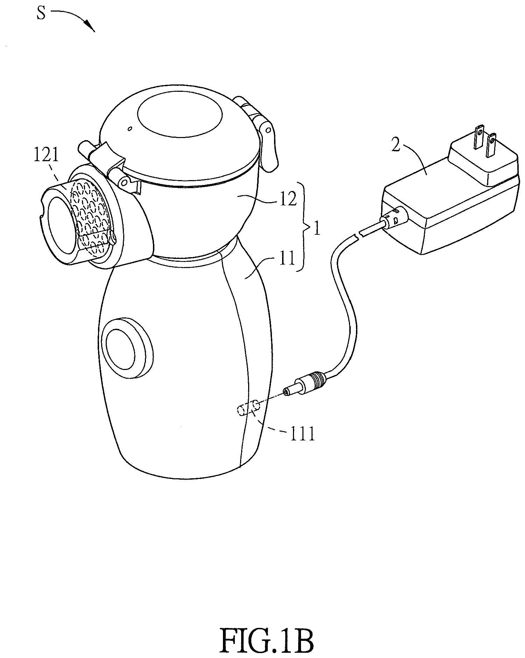

FIG. 1A is a block diagram of a nebulization system according to the first embodiment, and FIG. 1B is a schematic diagram showing the structure of the nebulization system in FIG. 1A. Referring to FIG. 1A and FIG. 1B, the nebulization system S can be applied to healthcare or beauty care. In the embodiment, the nebulization system is applied to drugs of medical use for example. In the embodiment, the nebulization system S includes a nebulizer 1 and a power adapter 2. The power adapter 2 is detachably connected to the nebulizer 1 and supplies power to the nebulizer 1 (can be detached from those after connection).

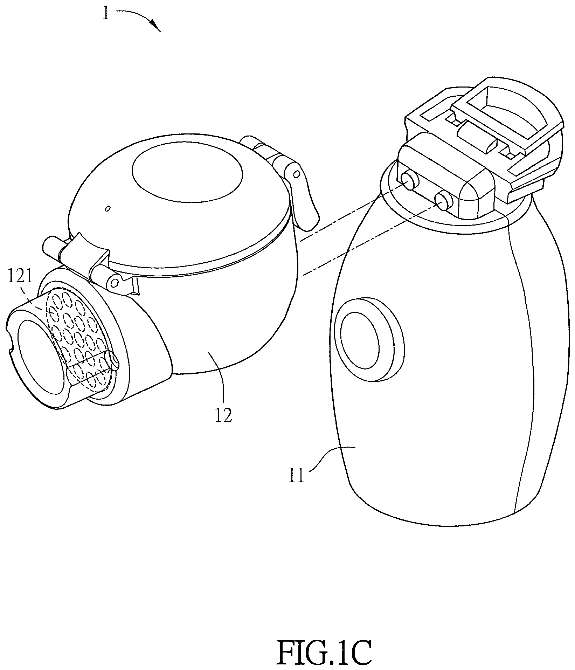

Please also referring to FIG. 1C, it is an exploded diagram showing the nebulizer. The nebulizer 1 includes a nebulization driving device 11 and a nebulization module 12. The nebulization module 12 provides an accommodation room for placing a liquid or drug storage. The nebulization driving device 11 connects to the nebulization module 12 and drives the nebulization module 12 to nebulize the liquid so as to generate spray or aerosol. In the embodiment, the nebulization driving device 11 and the nebulization module 12 can be separate and individual elements, and they can connect to each other for example by buckle or chute. Therefore, when the user wants to clean or wash the nebulization module 12, he can separate the nebulization driving device 11 and the nebulization module 12, and then merely clean the nebulization module 12 to avoid the electronic elements within the nebulization driving device 11 from damage due to damp. In other examples, the nebulization driving device 11 and the nebulization module 12 may be formed integrally.

The nebulization driving device 11 includes an input terminal 111, a battery unit 112, a switch unit 113, a voltage detection unit 114, a voltage adjustment unit 115, a driving unit 116, a PWM unit 117 and a control unit 118. It is noted that the PWM unit 117 is not necessary. In some embodiments where the PWM unit 117 is absent, the nebulizer 1 still keeps functions and operates well. In some embodiments with the PWM unit 117, it enhances the power management efficiency of the nebulization driving device 11 as described hereinafter.

The control unit 118 is coupled to the switch unit 113, the voltage detection unit 114, the voltage adjustment unit 115 and the PWM (pulse width modulation) unit 117 to receive signals from the above respective units or control the actions of the above respective units. The control unit 118 can be implemented with a digital circuit such as IC (integrated circuit) or an analog circuit. For example, IC can be micro-processor, MCU (micro control unit), FPGA (field-programmable gate array) or CPLD (complex programmable logic device), or ASIC (application-specific integrated circuit), which is not limited thereto. In the embodiment where the PWM unit 117 is absent, the control unit 118 is directly coupled to the driving unit 116 or the control unit 118 is indirectly coupled to the driving unit 116.

The input terminal 111 can be coupled to the power adapter 2 so that the power adapter 2 can supply power to the nebulization driving device 11. The input terminal 111 can be also coupled to the battery unit 112. If the nebulizer 1 is not coupled to mains electricity, the battery unit 112 can act as the power source of the nebulization driving device 11.

One terminal of the switch unit 113 is coupled to the input terminal 111, and the other terminal is coupled to the battery unit 112. The voltage detection unit 114 is coupled to the input terminal 111 to detect whether the voltage of the input terminal 111 is larger than a predefined value. In the embodiment, if the power adapter 2 is coupled to the input terminal 111 and supplies mains electricity, the voltage supplied by the power adapter 2 can be higher than the voltage supplied by the battery unit 112, and the predefined value is set between these two voltages. For example, the power adapter 2 supplies 5V and the battery unit 112 supplies 3V, the predefined value can be set 4V. Thus, if the voltage detection unit 114 detects that an input voltage of the input terminal is larger than the predefined value and then returns a detection signal to the control unit 118, the control unit 118 accordingly determines that the power adapter 2 is coupled to the input terminal 111. The power adapter 2 also supplies mains electricity to the nebulizer 1. Then the control unit 118 controls the switch unit 113 to cut off (or disconnect) so that the battery unit 112 cannot supply power to the input terminal 111 and cannot be charged by mains electricity. Thus, the battery unit 112 is protected and its usage life will not be negatively influenced.

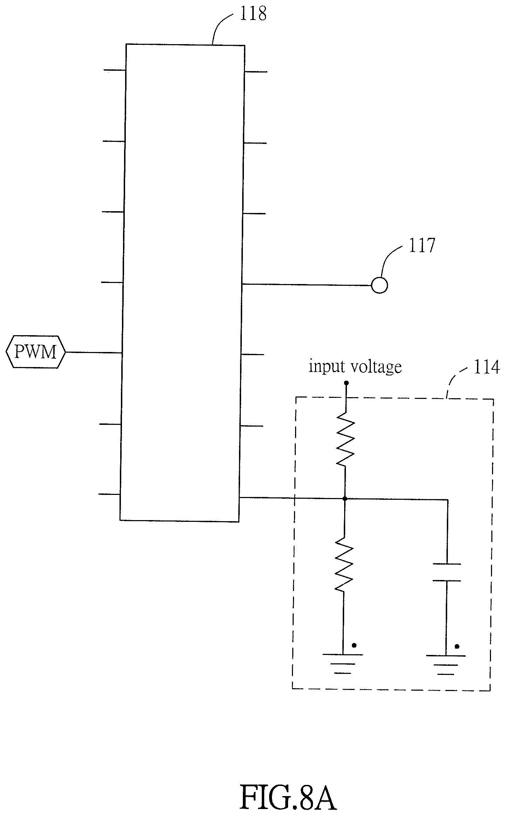

Subsequently, if the voltage detection unit 114 detects that an input voltage of the input terminal 111 is smaller than the predefined value and then returns a detection signal to the control unit 118, the control unit 118 accordingly determines that the power adapter 2 does not supply mains electricity to the nebulizer 1, for example the power adapter 2 is not coupled to the input terminal 111 or alternatively the power adapter 2 is coupled to the input terminal 111 but not coupled to mains electricity. At the moment, the control unit 118 controls the switch unit 113 to be conductible so as to utilize the battery unit 112 as the power source. For example, the circuit of the voltage detection unit 114 can refer to FIG. 8A.

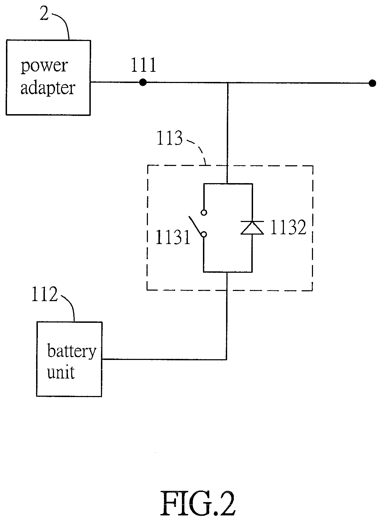

In details, referring to FIG. 2, it is a schematic diagram showing the circuit of the switch unit. The switch unit 113 can include a switch 1131 and a diode 1132. One terminal of the switch 1131 is coupled to the battery unit 112 and the anode of the diode 1132. Another terminal of the switch 1131 is coupled to the input terminal 111 and the cathode of the diode 1132. The other terminal of the switch 1131 is coupled to the control unit 118 as shown in FIG.1A. Here, the control unit 118 can control the switch 1131 to be cut off or conductible as above mentioned determination. Besides, in other embodiments, the switch unit 113 may be implemented with merely one switch 1131 or merely on diode 1132 which is not limited thereto.

The voltage adjustment unit 115 adjusts the input voltage received by the input terminal 111 to the operating voltage and outputs it to the driving unit 116. The input voltage can be provided by the power adapter 2 or the battery unit 112. In the embodiment, the voltage adjustment unit 115 can be a boost circuit, a buck circuit, or a boost-buck circuit for example, and it is not limited thereto.

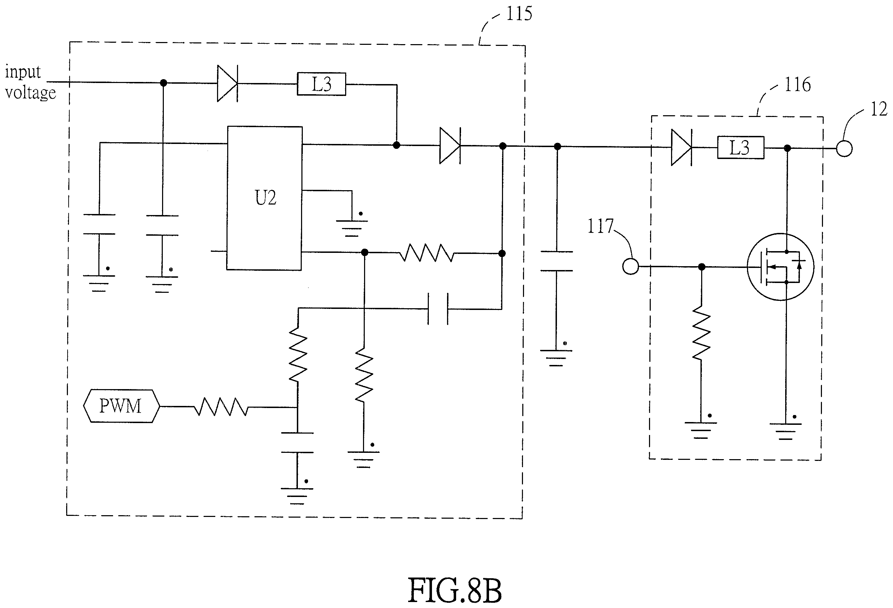

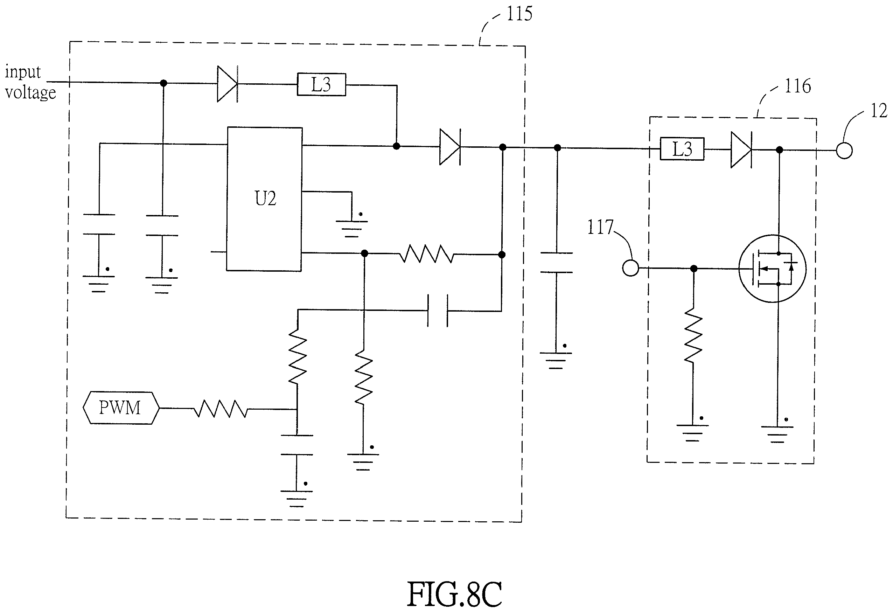

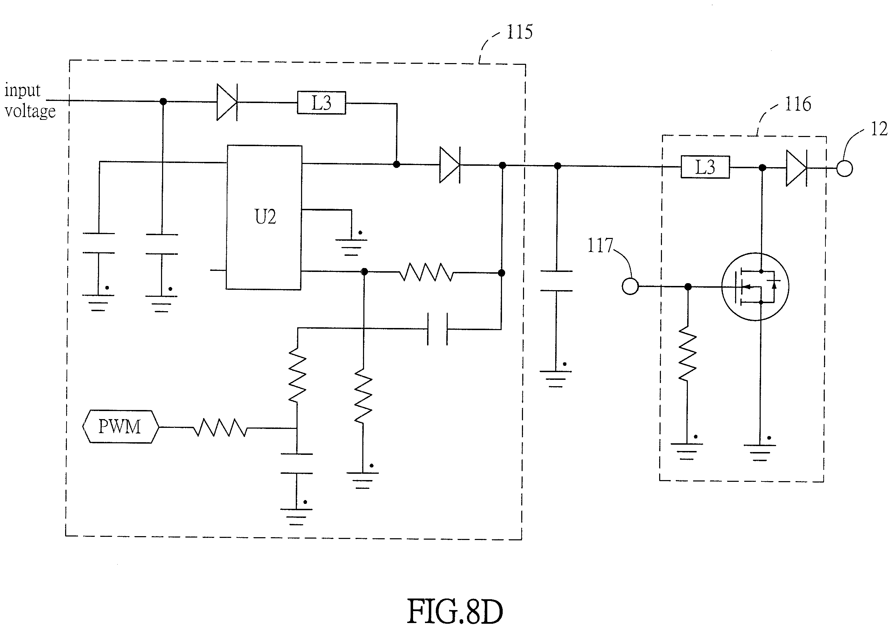

The driving unit 116 is coupled to the voltage adjustment unit 115, and drives the vibration unit 121 of the nebulization module 12 to vibrate according to the operation voltage of the voltage adjustment unit 115 so as to nebulize the medicated liquid to generate spray or aerosol. The vibration unit 121 can be piezoelectric material. The vibration is generated by applying voltage on the piezoelectric material so as to convert medicated liquid into spray or aerosol. The higher the applied voltage level, the larger the generated vibration and the more the spray quantity. For example, the circuit of the voltage adjustment unit 115 and the driving unit 116 can refer to FIG. 8B. The voltage adjustment unit 115 may be implemented by the power chip U2. Besides, other examples of the driving unit 116 may refer to FIG. 8C and FIG. 8D.

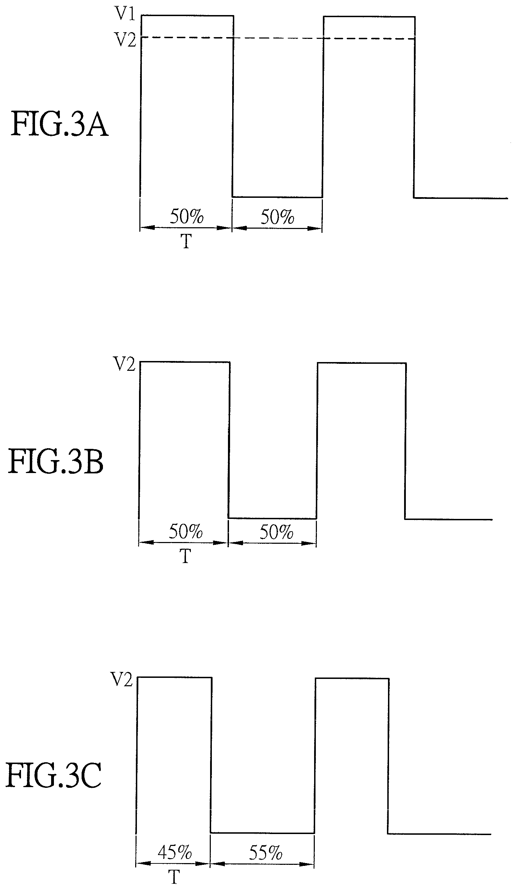

FIG. 3A is a waveform diagram showing the output voltage of the driving unit. Referring to FIG. 1A and FIG. 3A, in the embodiment, the control unit 118 can decide the operating voltage according to the power source. If supplied by mains electricity, the control unit 118 sets the nebulization driving device 11 in an ordinary driving mode (or first driving mode). If supplied by the battery unit 112, the control unit 118 sets the nebulization driving device 11 in an energy-saving driving mode (or second driving mode). In the ordinary driving mode, the voltage adjustment unit 115 can adjust the input voltage to the first operating voltage VI (for example 10V). In the energy-saving driving mode, the voltage adjustment unit 115 can adjust the input voltage to the second operating voltage V2 (for example 8V). The first operating voltage VI is larger than the second operating voltage V2. Therefore, in the energy-saving driving mode, the battery unit 112 can supplies lower power instead of the first operating voltage VI so as to extend supply time of the battery unit 112. Besides, the second operating voltage V2 can be 60% to 80% of the first operating voltage VI so that the treatment effect will not be influenced due to energy-saving.

FIG. 3B and FIG. 3C are waveform diagrams showing another output voltage of the driving unit. Referring to FIG. 1A and FIG. 3B, the PWM unit 117 is coupled to the driving unit 116 and generates a PWM signal to control the duty cycle T of the driving unit 116. For example, the duty cycle T can be 50%, namely, the periods for supplying drug and stopping supplying drug are equal to each other. In some embodiments, either the ordinary driving mode or the energy-saving driving mode, the PWM unit 117 may still generate the PWM signals which have the same duty cycle T. Namely, the duty cycle is not influenced by different driving modes. Referring to FIG. 1A and FIG. 3C, in other embodiments, the control unit 118 can control the PWM unit 117 to generate a PWM signal which has shorter duty cycle T in the energy-saving driving mode. For example, the duty cycle T is adjusted to 45% to save the power consumption of the battery unit 112 and prolong the usage time of the battery unit 112.

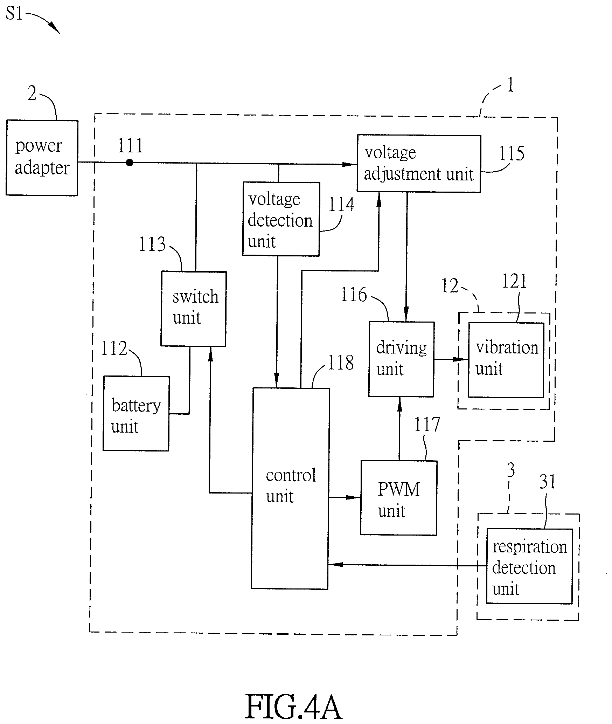

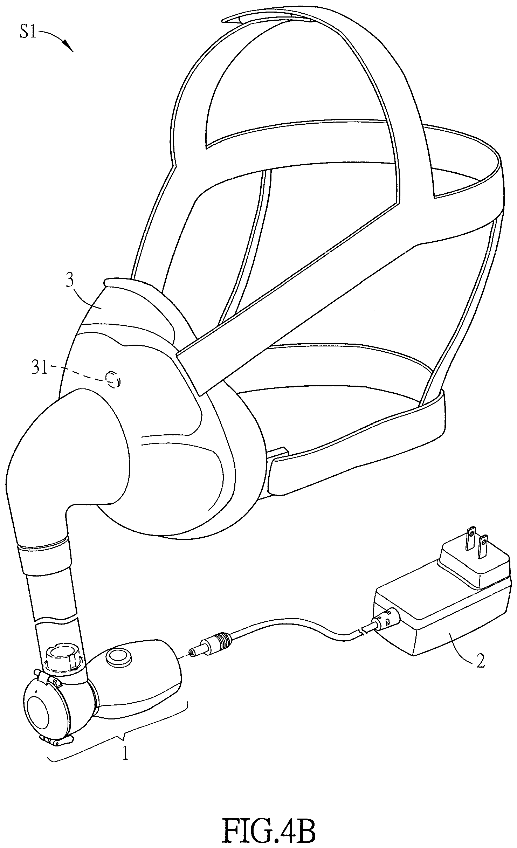

FIG. 4A is a block diagram of a nebulization system according to the second embodiment, and FIG. 4B is a schematic diagram showing the structure of the nebulization system in FIG. 4A. Referring to FIG. 4A and FIG. 4B, this embodiment is roughly similar to the first embodiment. The difference is that the nebulization system S1 in the embodiment further includes an auxiliary element 3. The auxiliary element 3 can be a face mask, mouthpiece, or catheter, and it is a face mask in the embodiment for example. The auxiliary element 3 has a respiration detection unit 31. The respiration detection unit 31 is coupled to the control unit 118. In the embodiment, the respiration detection unit 31 can detect the breath state of the user and send the detection result to the control unit 118, and the control unit 118 can accordingly control the driving unit 116 to act or not.

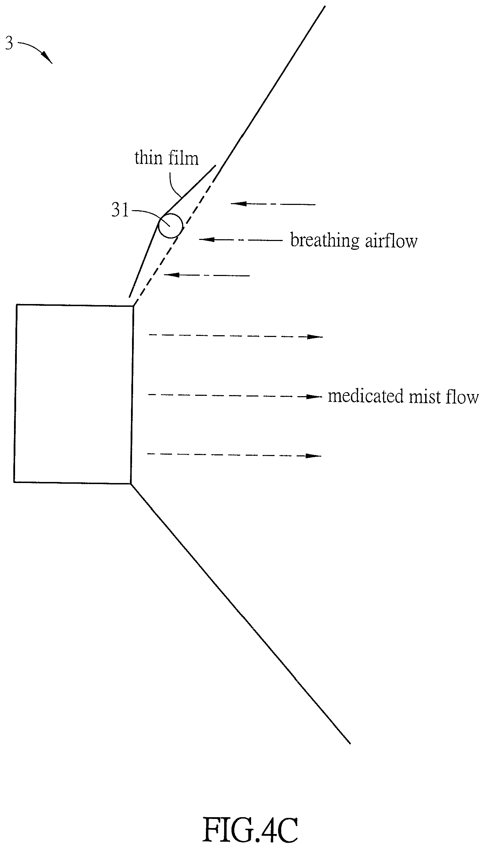

FIG. 4C is a schematic diagram showing the auxiliary element. Referring to FIG. 4A, FIG. 4B and FIG. 4C, in the embodiment, the respiration detection unit 31 is a micro switch. A thin film is disposed on the face mask (the auxiliary element 3), and the thin film is adjacent to the micro switch. As the user wears the face mask and breaths in for the spray treatment, the thin film may be drawn by the breathing airflow toward the user so as to push the micro switch. Thus, the micro switch may be actuated to transmit a signal to the control unit 118. Then, the control unit 118 accordingly controls the driving unit 116 to act so as to provide the medicated mist for the spray treatment. On the other hand, as the user breathes out, he blows the thin film away from the micro switch so that the micro switch is no longer extruded or pressed by the thin film and then be turned off. At the moment, because the control unit 118 does not receive the signal from the micro switch, it controls the driving unit 116 not to act so that the medicated mist is not provided for the user and it avoids that the user blows the medicated mist out and wastes the drug.

In some embodiments, the driving unit 116 may be controlled to act or not by some switches which set between the PWM unit 117 and the driving unit 116 and controlled by the control unit 118 to be conductible or cut off. Alternatively, some switches may be disposed between the control unit 118 and the PWM unit 117 and controlled by the control unit 118 similarly. They are not limited thereto.

It should be noted that although the respiration detection unit 31 in the embodiment is triggered by breathing in for example. For example, the respiration detection unit 31 can be also triggered by breathing out so as to stop the nebulizer 1 supplying drug, and they are not limited thereto.

Besides, in some embodiments, the respiration detection unit 31 can be an infrared detector or an audio detector. The infrared detector can detect variations or fluctuations of breathing airflow so as to determine whether the user is breathing in or breathing out. The audio detector can detect the sounds when the user breathes in or out so as to accordingly determine the breath state of the user.

It is noted that in the embodiment, the driving method by detecting breath to supply drug can be applied to the driving mode in the previous embodiment. For example, in the energy-saving driving mode, when the respiration detection unit 31 detects that the user breathes in, the control unit 118 can control the driving unit 116 to act either at the moment the input voltage of the driving unit 116 is at the second operating voltage V2 or at the moment the PWM unit 117 generates a PWM signal with 45% duty cycle. When the respiration detection unit 31 detects that the user breathes out, the control unit 118 controls the driving unit 116 to stop acting. Similarly, the ordinary driving mode can be also applied to the above embodiment, and it is not repeated here.

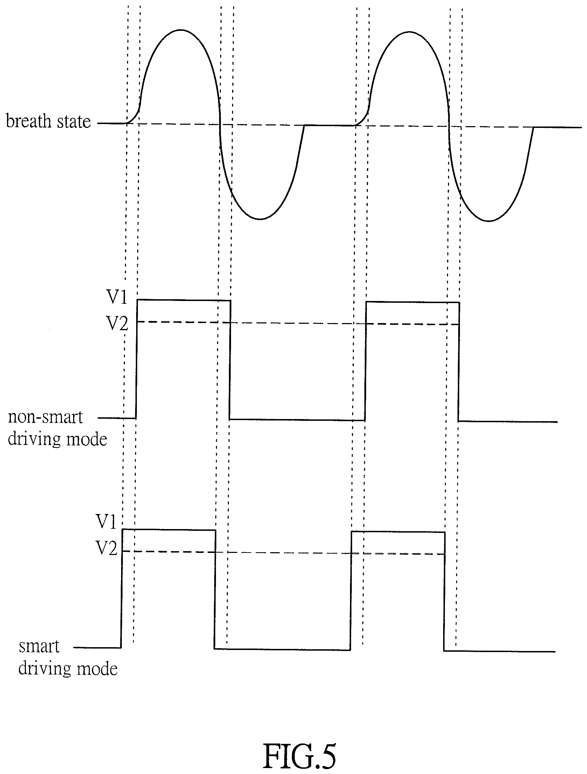

Besides, referring to FIG. 5, FIG. 5 is a waveform diagram showing the breath cycle of the user and the output voltage of the driving unit in smart driving mode. In the embodiment, the nebulization system S1 further provides a smart driving mode (or third driving mode). In the smart driving mode, the control unit 118 can control the actuation period and the stop period of the driving unit 116 so as to supply the nebulized liquid beforehand or synchronize the period of supplying the nebulized liquid and the period of breathing in. Furthermore, the control unit 118 can calculate the frequency and cycle of the user's breath and compute the average of the frequency and cycle of the breath so as to predict the later time period that the user breaths in. Therefore, the control unit 118 can control the driving unit 116 to act slightly earlier than the user breathes in, or supply drug synchronous with the period of breathing in of the user. The smart driving mode can solve the problem of delay supplying drug resulting from that after the respiration detection unit 31 detects that the user breathes in, the control unit 118 just receives the detection result and then controls the driving unit 116 to act.

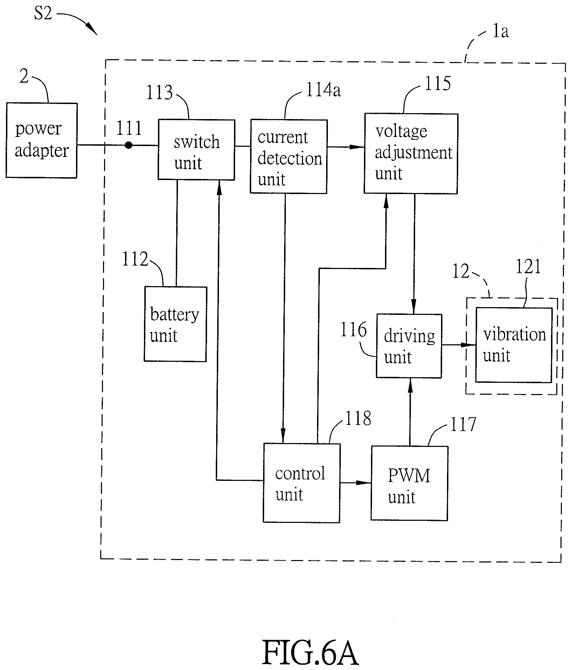

FIG. 6A is a block diagram of a nebulization system S2 according to the third embodiment. Referring to FIG. 6A, this embodiment is roughly similar to the first embodiment. The difference is that the nebulizer 1a in the embodiment does not utilize the voltage detection unit 114 to determine whether the power source is mains electricity or the battery unit 112. It utilizes the current detection unit 114a to determine the power source instead. In the embodiment, the current detection unit 114a is coupled to the input terminal 111, the voltage adjustment unit 115 and the control unit 118. As detecting a current, the current detection unit 114a may cause or convert the current signal to the control unit 118. While the battery unit 112 or the power adapter 2 supplies electric current to the current detection unit 114a, the current detection unit 114a can determine the power source according to whether the current quantity is larger than a predefined current value. For example, it is determined that the battery unit 112 supplies power as the current quantity is larger than the predefined current value, and it is determined that mains electricity supplies power if the current quantity is smaller than the predefined current value. In some embodiments, the current detection unit 114a for example can be a resistance, current transformer, or other elements capable of detecting current, which is not limited thereto. In other embodiments, the quantity of the input current can be measured based on the input voltage. For example, if the input voltage is supplied by the power adapter 2, the input voltage is 5V and the current is 0.25A; if the input voltage is supplied by a new battery (battery unit 112), the voltage is 3V and the current is 0.42A. Namely, under the power rating, the higher the input voltage is, the less the current is. Thus, according to the detected quantity of the current, it can be further determined whether the power source is mains electricity (the power adapter 2) or the battery unit 112. In one example, the power rating of the nebulizer 1 may be in a range between 0.8W to 2W, preferable 1.25W, so the nebulizer can achieve good performance and energy conservation.

Besides, in the embodiment, the input terminal 111 is coupled to the switch unit 113. Namely, the current provided by the power adapter 2 will flow through the switch unit 113 to the current detection unit 114a, and flow to the voltage adjustment unit 115. Because the elements and operations of the switch unit 113 can refer to the previous embodiment, they are not repeated here.

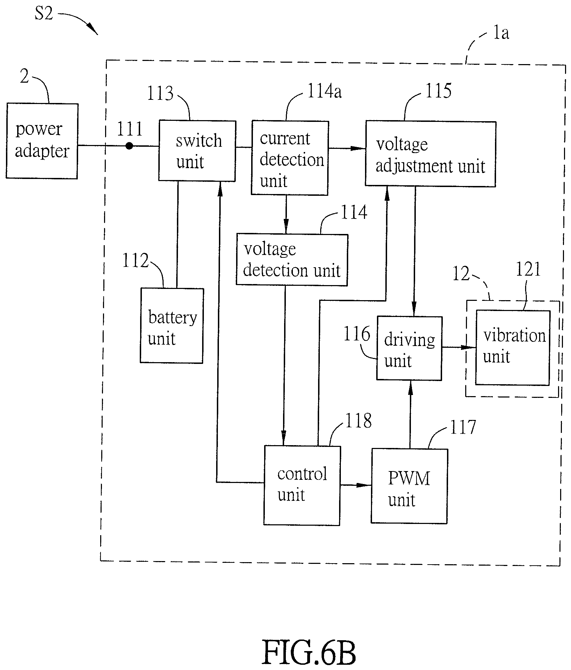

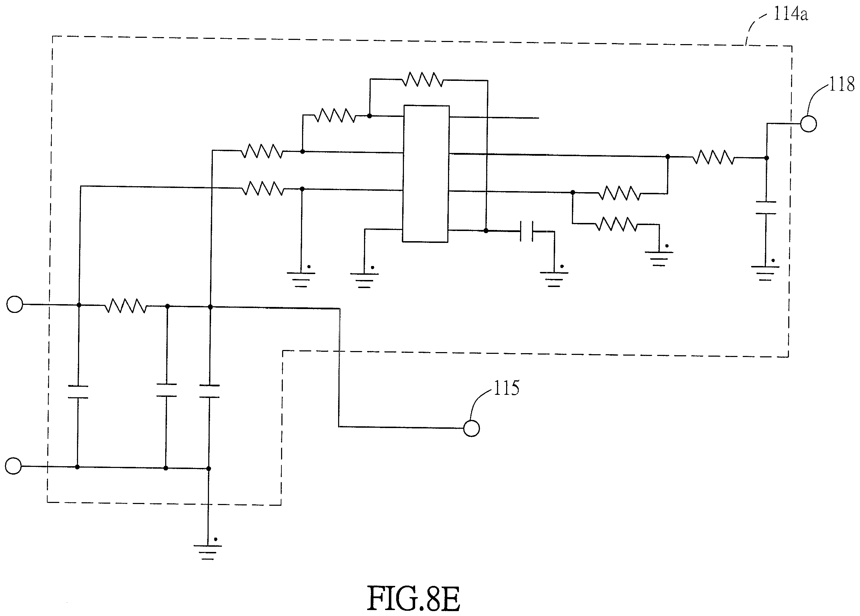

In some embodiments, both the voltage detection unit 114 and the current detection unit 114a are effective as shown in FIG. 6B for example. The voltage detection unit 114 is configured to determine the power source, and the current detection unit 114a is coupled to the input terminal 111, the voltage detection unit 114, the voltage adjustment unit 115, and the control unit 118 and is configured to detect an input current. Furthermore, the control unit 118 determines the power source according to the value of the input voltage detected by the voltage detection unit 114, and then calculates electric power according to a value of the input current and a value of the input voltage. If electric power determined and calculated by the control unit 118 is too high or too low, the control unit 118 would control the voltage adjustment unit 115 to adjust the output voltage (the operation voltage) to maintain power rating. In one example, after the voltage adjustment unit 115 outputs 10V as the first operation voltage while the input terminal 111 is coupled to the power adapter 2, if the control unit 118 determines electric power is lower than a predetermined value, such as 1.25W, the control unit 118 can control the voltage adjustment unit 115 to boost the first operation voltage, such as, to be 11V or 14V. Then, electric power would be raised to the predetermined value. In one embodiment, the circuit diagram of the current detection unit 114a is illustrated in FIG. 8E.

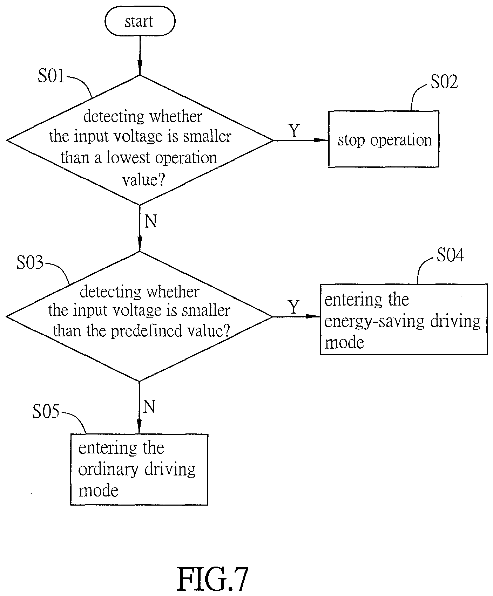

FIG. 7 is a flow chart showing steps of the driving method for a nebulization system according to an example not part of the invention. Referring to FIG. 1A, FIG. 1B and FIG. 7, the driving method in the embodiment is applied to the nebulization system such as the nebulization system S in the first embodiment mentioned above or the nebulization system S1 in the second embodiment mentioned above. Here, it is applied to the nebulization system S in the first embodiment for example. Because the elements and the operations of the nebulization system S can refer to the above descriptions, they are not repeated here. The driving method in the embodiment includes following steps of: detecting whether the imput voltage is smaller than a lowest operation value (S01); if YES, stop operation (S02); if NO, detecting whether the input voltage is smaller than the predefined value (S03); if the input voltage is smaller than the predefined value, entering the energy-saving driving mode (S04); if the input voltage is larger than the predefined value, entering the ordinary driving mode S05.

In step S01, if the nebulizer 1 does not connect to mains electricity and the battery unit 112 contains low quantity of electricity, the voltage detection unit 114 detects that the input voltage is inadequate to operate the nebulizer 1 even in the energy-saving driving mode. In the embodiment, the lowest operation value may be 1.8V. Thus, the control unit 118 will stop the operation or action of the driving unit 116 (step S02) as the input voltage is smaller than 1.8V. Otherwise, if the input voltage is adequate to trigger the nebulizer 1, step S03 is performed to determine whether the input voltage is smaller than the predefined value or not.

In step S04, in the energy-saving driving mode, the battery unit 112 supplies power and the input voltage of the driving unit 116 is the second operating voltage. In the embodiment, the PWM unit 117 can be utilized to reduce the duty cycle T of the driving unit 116 so as to further decrease the power consumption of the battery unit 112 and prolong the usage time of the battery unit 112.

In step S05, in the ordinary mode, mains electricity supplies power, the input voltage of the driving voltage is the first operating voltage, and the first operating voltage is larger than the second operating voltage. Thus, the power can be continuously supplied to enhance the spray treatment. Besides, the control unit 118 can further turn off the switch unit 113 so that the battery unit 112 will not supply power and will not be charged by mains electricity.

As mentioned above, in some embodiments, the nebulization system includes a respiration detection module and a spray adjustment module. The respiration detection module detects a breath state of a user to generate a detection result. The detection result can represent the breath rhythm of the user, for example a breathing in state or a breathing out state of the user at the moment, or an oncoming breathing in state or an oncoming breathing out state of the user. The spray adjustment module adjusts the time of generating the spray or the amount of generated spray by the nebulization module 12. Preferably, under control by the spray adjustment module, the spray of the nebulization module 12 is generated or outputted following the breath rhythm of the user. For example, the nebulization module 12 is controlled by the spray adjustment module not to generate the spray when the user breathes out. The respiration detection module can include the detection unit 31 mentioned above. The spray adjustment module can include the control unit 118 and the driving unit 116 mentioned above. In addition, the spray adjustment module can further include other elements of the nebulization driving device 11.

As mentioned above, the voltage or current of the input terminal is detected to accordingly determine whether the nebulizer is supplied with mains electricity or battery. Moreover, the driving mode is adjusted according to the power source so as to keep better performance and battery life. In some embodiments, the breath detection unit is utilized to detect the breath state of the user so that the nebulizer can adjust the drug supply with the user's breath rhythm so as to avoid a waste of medicated liquid.

Although the invention has been described with reference to specific embodiments, this description is not meant to be construed in a limiting sense. Various modifications of the disclosed embodiments, as well as alternative embodiments, will be apparent to persons skilled in the art. It is, therefore, contemplated that the appended claims will cover all modifications that fall within the true scope of the invention.

## Claims

1. A nebulization system, comprising:
a power adapter (2); and
a nebulizer (1) adapted to be detachably connected to the power adapter (2), comprising:
a nebulization module (12) having an accommodation room for placing a liquid; and
a nebulization driving device (11) connecting to the nebulization module (12) and comprising:
a battery unit (112);
an input terminal (111) coupled to the power adapter (2) and the battery unit (112);
**characterized by**
a voltage detection unit (114) coupled to the input terminal (111) to detect whether an input voltage of the input terminal (111) is larger than a predefined value, wherein the predefined value is set larger than a voltage outputted from the battery unit (112);
a voltage adjustment unit (115) coupled to the input terminal (111);
a control unit (118) coupled to the voltage detection unit (114) and the voltage adjustment unit (115), wherein the control (118) unit controls the voltage adjustment unit (115) to adjust the input voltage to a first operating voltage as an output voltage if the input voltage is larger than the predefined value, the control unit (118) controls the voltage adjustment unit (115) to adjust the input voltage to a second operating voltage as an output voltage if the input voltage is smaller than the predefined value, and the first operating voltage is larger than the second operating voltage; and
a driving unit (116) coupled to the voltage adjustment unit (115), and driving the nebulization module (12) to nebulize the liquid according to the output voltage of the voltage adjustment unit (115).

2. The nebulization system of claim 1, wherein the nebulization driving device (11) further comprises a PWM unit (117) coupled to the driving unit (116) and generating a PWM signal to control a duty cycle of the driving unit (116) to drive the nebulization module (12) to nebulize the liquid.

3. The nebulization system of claim 1 or 2, wherein the nebulization driving device (11) further comprises a switch unit (113) having one terminal coupled to the input terminal (111) and having the other terminal coupled to the battery unit (112), and the control unit (118) controls the switch unit (113) to cut off so the battery unit (112) does not supply power to the input terminal (111) if the input voltage is larger than the predefined value.

4. The nebulization system of claim 3, wherein the switch unit (113) comprises a switch (1131) and a diode (1132), one terminal of the switch (1131) is coupled to the battery unit (112) and the anode of the diode (1132), another terminal of the switch (1131) is coupled to the input terminal (111) and the cathode of the diode (1132), and the other terminal of the switch (1131) is coupled to the control unit (118).

5. The nebulization system of one of claims 1-4, further comprising an auxiliary element (3) including a respiration detection unit (31) coupled to the control unit (118) to detect the breath state of a user, wherein the control unit (118) controls the driving unit (116) to act if the user breathes in, and the control unit (118) controls the driving unit (116) to stop acting if the user breathes out.

6. The nebulization system of claim 5, wherein the respiration detection unit (31) is a micro switch, an infrared detector or an audio detector.

7. The nebulization system of one of claims 1-6, wherein the predefined value is smaller than a voltage outputted from the power adapter.

8. The nebulization system of one of claims 1-7, further comprising a current detection unit (114a) coupled to the input terminal (111) to detect an input current corresponding to the input voltage, wherein the control unit (118) calculates electric power according to the input current and the input voltage.

## Patentansprüche

1. Zerstäubungssystem mit:
einem Energieadapter (2); und
einem Zerstäuber (1), der lösbar mit dem Energieadapter (2) verbindbar ist, mit:
einem Zerstäubungsmodul (12) mit einem Aufnahmeraum zum Vorsehen einer Flüssigkeit; und
einer Zerstäubungsantriebsvorrichtung (11), welche mit dem Zerstäubungsmodul (12) verbunden ist und aufweist:
eine Batterieeinheit (112);
einen Eingangsanschluss (111), der mit dem Energieadapter (2) und der Batterieeinheit (112) gekoppelt ist;
**gekennzeichnet durch**
eine Spannungserkennungseinheit (114), die mit dem Eingangsanschluss (111) gekoppelt ist, um zu erkennen, ob eine Eingangsspannung des Eingangsanschlusses (111) größer als ein vordefinierter Wert ist, wobei der vordefinierte Wert größer als eine von der Batterieeinheit (112) ausgegebene Spannung eingestellt ist;
eine Spannungseinstelleinheit (115), die mit dem Eingangsanschluss (111) gekoppelt ist;
eine Steuereinheit (118), die mit der Spannungserkennungseinheit (114) und der Spannungseinstelleinheit (115) gekoppelt ist, wobei die Steuereinheit (118) die Spannungseinstelleinheit (115) zum Einstellen der Eingangsspannung auf eine erste Betriebsspannung als eine Ausgangsspannung steuert, wenn die Eingangsspannung höher als der vordefinierte Wert ist, und die Steuereinheit (118) die Spannungseinstelleinheit (115) zum Einstellen der Eingangsspannung auf eine zweite Betriebsspannung als eine Ausgangsspannung steuert, wenn die Eingangsspannung niedriger als der vordefinierte Wert ist, und die erste Betriebsspannung ist höher als die zweite Betriebsspannung; und
eine Antriebseinheit (116), die mit der Spannungseinstelleinheit (115) gekoppelt ist und das Zerstäubungsmodul (12) antreibt, um die Flüssigkeit entsprechend der Ausgangsspannung der Spannungseinstelleinheit (115) zu zerstäuben.

2. Zerstäubungssystem nach Anspruch 1, bei welchem die Zerstäubungsantriebsvorrichtung (11) ferner eine PWM-Einheit (117) aufweist, die mit der Antriebseinheit (116) gekoppelt ist und ein PWM-Signal erzeugt, um ein Tastverhältnis der Antriebseinheit (116) zu steuern, um das Zerstäubungsmodul (12) zum Zerstäuber der Flüssigkeit anzutreiben.

3. Zerstäubungssystem nach Anspruch 1 oder 2, bei welchem die Zerstäubungsantriebsvorrichtung (11) ferner eine Schalteinheit (113) aufweist, deren einer Anschluss mit dem Eingangsanschluss (111) gekoppelt ist und deren anderer Anschluss mit der Batterieeinheit (112) gekoppelt ist, und wobei die Steuereinheit (118) die Schalteinheit (113) derart steuert, dass sie abschaltet, so dass sie Batterieeinheit (112) keine Energie an den Eingangsanschluss (111) liefert, wenn die Eingangsspannung höher als der vordefinierte Wert ist.

4. Zerstäubungssystem nach Anspruch 3, bei welchem die Schalteinheit (113) einen Schalter (1131) und eine Diode (1132) aufweist, wobei ein Anschluss des Schalters (1131) mit der Batterieeinheit (112) und der Anode der Diode (1132) gekoppelt ist, ein anderer Anschluss des Schalters (1131) mit dem Eingangsanschluss (111) und der Kathode der Diode (1132) gekoppelt ist, und der andere Anschluss des Schalters (1131) mit der Steuereinheit (118) gekoppelt ist.

5. Zerstäubungssystem nach einem der Ansprüche 1 - 4, ferner mit einem Zusatzelement (3), das eine Atmungserkennungseinheit (31) aufweist, welche mit der Steuereinheit (118) gekoppelt ist, um den Atemzustand eines Benutzers zu erkennen, wobei die Steuereinheit (118) die Antriebseinheit (116) steuert, um zu agieren, wenn der Benutzer einatmet, und die Steuereinheit (118) die Antriebseinheit (116) steuert, um ihre Aktion zu beenden, wenn der Benutzer ausatmet.

6. Zerstäubungssystem nach Anspruch 5, bei welchem die Atmungserkennungseinheit (31) ein Mikroschalter, ein Infrarotdetektor oder ein Audiodetektor ist.

7. Zerstäubungssystem nach einem der Ansprüche 1 - 6, bei welchem der vordefinierte Wert niedriger als eine von dem Energieadapter ausgegebene Spannung ist.

8. Zerstäubungssystem nach einem der Ansprüche 1 - 7, ferner mit einer Stromerkennungseinheit (114a), die mit dem Eingangsanschluss (111) gekoppelt ist, um einen der Eingangsspannung entsprechenden Eingangsstrom zu erkennen, wobei die Steuereinheit (118) die elektrische Energie entsprechend dem Eingangsstrom und der Eingangsspannung berechnet.

## Revendications

1. Système de nébulisation, comprenant :
un adaptateur d'alimentation (2) ; et
un nébuliseur (1) adapté pour être relié de manière amovible à l'adaptateur d'alimentation (2), et comprenant :
un module de nébulisation (12) pourvu d'un espace de logement pour placer un liquide ; et
un dispositif d'entraînement de nébulisation (11) se raccordant au module de nébulisation (12) et comprenant :
un ensemble batterie (112) ;
une borne d'entrée (111) couplée à l'adaptateur d'alimentation (2) et à l'ensemble batterie (112) ;
**caractérisé par**
une unité de détection de tension (114) couplée à la borne d'entrée (111) pour détecter si une tension d'entrée de la borne d'entrée (111) est supérieure à une valeur prédéfinie, la valeur prédéfinie étant fixée à une valeur supérieure à une tension délivrée en sortie de l'ensemble batterie (112) ;
une unité de réglage de tension (115) couplée à la borne d'entrée (111) ;
une unité de commande (118) couplée à l'unité de détection de tension (114) et à l'unité de réglage de tension (115), laquelle unité de commande (118) commande l'unité de réglage de tension (115) pour régler la tension d'entrée à une première tension de service en tant que tension de sortie si la tension d'entrée est supérieure à la valeur prédéfinie, et l'unité de commande (118) commande l'unité de réglage de tension (115) pour régler la tension d'entrée à une seconde tension de service en tant que tension de sortie si la tension d'entrée est inférieure à la valeur prédéfinie, la première tension de service étant supérieure à la seconde tension de service ; et
une unité d'entraînement (116) couplée à l'unité de réglage de tension (115) et entraînant le module de nébulisation (12) pour nébuliser le liquide en fonction de la tension de sortie de l'unité de réglage de tension (115).

2. Système de nébulisation selon la revendication 1, dans lequel le dispositif d'entraînement de nébulisation (11) comprend en outre une unité de modulation PWM (117) couplée à l'unité d'entraînement (116) et générant un signal PWM pour commander un facteur de marche de l'unité d'entraînement (116) qui amène le module de nébulisation (12) à nébuliser le liquide.

3. Système de nébulisation selon la revendication 1 ou 2, dans lequel le dispositif d'entraînement de nébulisation (11) comprend en outre une unité de commutation (113) dont une borne est couplée à la borne d'entrée (111) et l'autre borne est couplée à l'ensemble batterie (112), et dans lequel l'unité de commande (118) commande l'unité de commutation (113) pour opérer une coupure afin que l'ensemble batterie (112) n'alimente pas la borne d'entrée (111) si la tension d'entrée est supérieure à la valeur prédéfinie.

4. Système de nébulisation selon la revendication 3, dans lequel l'unité de commutation (113) comprend un commutateur (1131) et une diode (1132), une borne du commutateur (1131) est couplée à l'ensemble batterie (112) et à l'anode de la diode (1132), une autre borne du commutateur (1131) est couplée à la borne d'entrée (111) et à la cathode de la diode (1132), et l'autre borne du commutateur (1131) est couplée à l'unité de commande (118).

5. Système de nébulisation selon l'une des revendications 1 à 4, comprenant en outre un élément auxiliaire (3) incluant une unité de détection de respiration (31) couplée à l'unité de commande (118) afin de détecter l'état de respiration d'un utilisateur, dans lequel l'unité de commande (118) commande à l'unité d'entraînement (116) d'agir si l'utilisateur inspire et l'unité de commande (118) commande à l'unité d'entraînement (116) de cesser d'agir si l'utilisateur expire.

6. Système de nébulisation selon la revendication 5, dans lequel l'unité de détection de respiration (31) est un microrupteur, un détecteur infrarouge ou un détecteur audio.

7. Système de nébulisation selon l'une des revendications 1 à 6, dans lequel la valeur prédéfinie est inférieure à une tension délivrée en sortie de l'adaptateur d'alimentation.

8. Système de nébulisation selon l'une des revendications 1 à 7, comprenant en outre une unité de détection de courant (114a) couplée à la borne d'entrée (111) afin de détecter un courant d'entrée correspondant à la tension d'entrée, l'unité de commande (118) calculant l'énergie électrique en fonction du courant d'entrée et de la tension d'entrée.
